# EUROPÄISCHE PATENTSCHRIFT

(11) **EP 1 131 275 B1**
(45) Veröffentlichungstag und Bekanntmachung des Hinweises auf die Patenterteilung: **17.09.2003**
(21) Anmeldenummer: 99956020.4
(22) Anmeldetag: 19.11.1999
(51) Int. Cl.: C07C 51/265, C07C 63/16, C07D 307/89

(54) **VERFAHREN ZUR HERSTELLUNG VON PHTHALSÄUREANHYDRID**
METHOD OF PRODUCING PHTHALIC ACID ANHYDRIDE
PROCEDE DE PREPARATION D'ANHYDRIDE D'ACIDE PHTALIQUE

(30) Priorität: 20.11.1998 DE 19853675; 27.11.1998 DE 19854892
(43) Veröffentlichungstag der Anmeldung: 12.09.2001
(73) Patentinhaber: BASF AKTIENGESELLSCHAFT, 67056 Ludwigshafen (DE)
(72) Erfinder: REUTER, Peter, D-68199 Mannheim (DE); VOIT, Guido, D-67251 Freinsheim (DE)
(74) Vertreter: Kinzebach, Werner, Dr.
(86) Internationale Anmeldenummer: EP9908911
(87) Internationale Veröffentlichungsnummer: WO00031013

(56) Entgegenhaltungen:
- US-A- 2 574 512
- CHEMICAL ABSTRACTS, vol. 100, no. 5, 30. Januar 1984 (1984-01-30) Columbus, Ohio, US; abstract no. 34218a, GLUKHOVSKII N G ET AL: "Effect of certain impurities in industrial-grade air on the oxidation of o-xylene to phthalic anhydride" Seite 422; XP002131514 & NEFTEPERERAB. NEFTEKHIM. (MOSCOW), Bd. 10, 1983, Seiten 17-20,

## Beschreibung

Die vorliegende Erfindung betrifft-ein Verfahren zur Herstellung von Phthalsäureanhydrid durch katalytische Oxidation von o-Xylol in Gegenwart von Schwefeldioxid in der Gasphase.

Phthalsäureanhydrid wird großtechnisch durch katalytische Oxidation von o-Xylol in der Gasphase hergestellt. Hierzu wird ein o-Xylol und Sauerstoff enthaltender Gasstrom bei erhöhter Temperatur über einen heterogenen Katalysator geleitet. Der Katalysator befindet sich in der Regel in einem Festbettröhrenreaktor. Als Katalysator sind für dieses Verfahren insbesondere Trägerkatalysatoren geeignet, die aus einem inerten Träger und einer darauf in dünner Schicht aufgebrachten katalytischen wirksamen Masse aus beispielsweise Vanadinpentoxid und Titandioxid bestehen. Solche Katalysatoren sind z.B. in der DE-A-1442590 beschrieben. Die Reaktorrohre sind in der Regel mit einer Salzschmelze umgeben, in der man eine Temperatur von 350-420°C einhält. Die aus dem Reaktor austretenden Dämpfe werden abgekühlt und kondensiert.

Es ist bekannt, dem Gasstrom zur Aktivitätssteigerung der eingesetzten Katalysatoren, insbesondere nach längerer Betriebsdauer der Katalysatoren, katalytische Mengen von Schwefeldioxid zuzusetzen (siehe Nikolov et al., Catal. Rev.-Sci. Eng. 33 (1991) 319).

O-Xylol wird großtechnisch aus einer BTX-Fraktion erhalten, die durch Flüssigextraktion geeigneter Einsatzmaterialien, z.B. katalytischen Reformaten oder hydrierten Pyrrolyseölen, gewonnen wird. Als Extraktionslösemittel werden überwiegend N-Acyl-Verbindungen, wie insbesondere N-Formylmorpholin, eingesetzt. Aus diesem Grund enthält das im Handel erhältliche technische o-Xylol mehr oder weniger große Mengen dieser N-Acyl-Verbindungen.

Bei der technischen Durchführung des eingangs beschriebenen Verfahrens zur Herstellung von Phthalsäureanhydrid hat es sich gezeigt, dass sich in der Anlage an Stellen, an denen flüssiges oder gasförmiges o-Xylol mit einem Schwefeldioxid enthaltenden Luftstrom bei erhöhter Temperatur in Kontakt kommt, feste Ablagerungen bilden. Diese Ablagerungen sind aus verschiedenen Gründen unerwünscht, insbesondere weil es zu einem Verstopfen von Rohrleitungen, zu einem Verdichten des Katalysatorbetts oder zu einer thermischen Isolierung von Wärmetauschflächen für die Verdampfung von flüssigem o-Xylol kommt.

Der vorliegenden Erfindung liegt daher die Aufgabe zugrunde, das eingangs beschriebene Verfahren so weiterzubilden, dass die beschriebenen Ablagerungen vermieden werden.

Erfindungsgemäß wird diese Aufgabe durch ein Verfahren zur Herstellung von Phthalsäureanhydrid durch Oxidation von technischem o-Xylol in der Gasphase gelöst, wobei man einen o-Xylol, Sauerstoff und Schwefeldioxid enthaltenden Gasstrom bei erhöhter Temperatur über einen schwermetallhaltigen Katalysator leitet, das dadurch gekennzeichnet ist, dass der Gehalt des Gasstroms an N-Formylmorpholin, vorzugsweise der Gesamtgehalt an N-Acyl-Verbindungen, weniger als 50 ppm, insbesondere weniger als 10 ppm, bezogen auf das Gewicht an o-Xylol, beträgt. Der Gasstrom kann z.B., 5 bis weniger als 50 ppm oder 5 bis weniger als 10 ppm N-Acyl-verbindungen, bezogen auf o-Xylol, enthalten.

Vorzugsweise enthält der Gasstrom 50 bis 160 g, insbesondere 60-120 g, o-Xylol pro m³ und 0,01 bis 5 Gew.-%, insbesondere 0,1 bis 1,5 Gew.-%, bezogen auf o-Xylol Schwefeldioxid in einem 1 bis 100 Vol.-%, insbesondere 2 bis 50 Vol.-%, Sauerstoff enthaltenden Gas(gemisch), wie insbesondere Luft.

Unter N-Acyl-Verbindungen werden niedermolekulare stickstoffhaltige organische Verbindungen verstanden, die am Stickstoffatom eine Acylgruppe tragen. Es kann sich dabei um offenkettige Amide oder Lactame handeln. Sie weisen im Allgemeinen ein Molekulargewicht von weniger als 150, in der Regel von 70 bis 150, auf. Typische Vertreter sind z.B. N-Methylpyrrolidon, N-Formylmorpholin oder N,N-Dimethylformamid.

In den Spezifikationen der Hersteller von o-Xylol sind N-Acyl-Verbindungen üblicherweise nicht berücksichtigt. Es sind daher besondere Maßnahmen zu treffen, um die Bildung der erwähnten Ablagerungen zu verhindern.

Die Kontrolle des Gehaltes des Gasstroms an N-Acyl-Verbindungen erfolgt zweckmäßigerweise dadurch, dass man vor der Umsetzung in dem technischen o-Xylol den Gehalt an N-Acyl-Verbindungen bestimmt und,
a) wenn der Gehalt kleiner als 50 ppm ist, bezogen auf o-Xylol, das technische o-Xylol zur Umsetzung verwendet, oder
b) wenn der Gehalt größer als oder gleich 50 ppm ist, bezogen auf o-Xylol,
   i) das technische o-Xylol aus dem Verfahren ausschleust, oder
   ii) den Gehalt des technischen o-Xylols an N-Acyl-Verbindungen auf weniger als 50 ppm, bezogen auf o-Xylol, ein-stellt und das so erhaltene technische o-Xylol zur Umsetzung verwendet.

Die Bestimmung des Gehalts an N-Acylverbindungen kann z.B. mittels Gaschromatographie, gegebenenfalls unter Zuhilfenahme von geeigneten Referenzsubstanzen, erfolgen. Zur-Einstellung eines niedrigen Gehaltes an N-Acyl-Verbindungen sind z.B. Destillationsverfahren geeignet.

Im erfindungsgemäßen Verfahren sind die Katalysatoren geeignet, die üblicherweise zur Luftoxidation von o-Xylol eingesetzt werden. Diese sind in der Regel Katalysatoren auf Vanadinpentoxidbasis. Die Vanadinkatalysatoren können mit unterschiedlichen anderen Elementen des Periodensystems dotiert sein, z.B. mit Cäsium (siehe WO 98/37965), Phosphor (DE-OS 1769998) bzw. mit Rubidium und/oder Cäsium (DE-PS 2436009 und DE-PS 2547624). Die Katalysatoren werden in der Regel auf Trägern eingesetzt. Der Träger hat z.B. die Gestalt einer Kugel oder vorzugsweise Ringform. Er besteht z.B. aus gesinterten oder geschmolzenen Silikaten, Porzellan, Tonerde, Siliciumcarbid oder Quarz. Die katalytisch aktive Masse, die den Träger vorzugsweise mit einer Schichtdicke von 0,05-1 mm überzieht, enthält z.B. 1-30 Gew.-% Vanadinpentoxid und 70-99 Gew.-% Titandioxid. Sie kann gegebenenfalls noch geringe Mengen, z.B. bis zu 5 Gew.-%, bezogen auf die katalytische Masse, an Antimon, Zirkon oder Zinn, Phosphor, Rubidium oder Cäsium, z.B. in Form ihrer Oxide enthalten. Die aktive Masse macht etwa 3-50 Gew.-% des fertigen Trägerkatalysators aus. Der Katalysator ist in der Regel in mehrere Röhren eines Röhrenreaktors eingefüllt.

Das erfindungsgemäße Verfahren wird zweckmäßigerweise wie folgt durchgeführt: Die vorzugsweise staubfreie, filtrierte Luft wird in einem Verdichter komprimiert. Diese verdichtete Luft wird in einem Luftvorwärmer, beispielsweise mittels Dampf, aufgeheizt. Anschließend wird in diesen Heißluftstrom o-Xylol eingedüst. Das eingedüste o-Xylol verdampft und verteilt sich im Luftstrom. Die Beladung des o-Xylols pro Kubikmeter Luft ist variabel. Sie kann unterhalb der Explosionsgrenze (44 g/m³) liegen. Es sind jedoch auch Beladungen von z.B. 44-100 g o-Xylol pro m³ Luft möglich. Dieses Gasgemisch, dem eine katalytische Menge SO₂, gewöhnlich 0,1 bis 1,5 Gew.-%, bezogen auf o-Xylol, zudosiert wird, wird in den Kopf des Reaktors geleitet. Dies wird auf die Rohre des Röhrenreaktors verteilt und strömt über den Katalysator. Die entstehende Reaktionswärme wird über ein geschmolzenes Salzbad, z.B. aus einem Gemisch von Kaliumnitrat und Natriumnitrit, als Wärmeübertragungsmedium abgeführt, das im Allgemeinen auf 300 bis 450°C thermostatisiert ist. Die Kühlung des heißen Salzes kann z.B. durch Erzeugung von Hochdruckdampf erfolgen. Vorteilhaft kann die Gasphasenoxidation so durchgeführt werden, dass man zwei oder mehr Zonen, vorzugsweise zwei Zonen, der im Reaktionsrohr befindlichen Katalysatorschüttung auf unterschiedliche Reaktionstemperaturen thermostatisiert, wobei beispielsweise Reaktoren mit getrennten Salzbädern, wie sie z.B. in der DE-A 2201525 oder DE-A 2830765 beschrieben sind, eingesetzt werden können. Dabei kann, wie in der DE-A 4013051 beschrieben, die zur Eintrittsstelle des Gasstroms gelegene Reaktionszone, die im Allgemeinen 25 bis 75 Vol.-% des gesamten Katalysatorvolumens umfasst, auf eine um 1 bis 20°C, vorzugsweise 1 bis 10°C und insbesondere um 2 bis 8°C höhere Reaktionstemperatur als die zum Gasaustritt gelegene Reaktionszone thermostatisiert werden.

Aus den Produktgasen wird das gebildete Phthalsäureanhydrid vorzugsweise durch Desublimation fest abgeschieden. Hierzu sind alternierend arbeitende Schmelzkondensatoren besonders geeignet. Dabei wird das gekühlte Produktgas in eine Gruppe von Abscheidern geleitet. Jeder einzelne dieser Kondensatoren arbeitet in einem Aufheiz- und Kühl- bzw. Schmelz- und Beladungszyklus. Befindet sich ein Abscheider in der Beladungsphase, so desublimiert Phthalsäureanhydrid auf den Kühlflächen. Die Kühlflächen werden von einem gekühlten Wärmeübertragungsmittel durchflossen. Bei einem bestimmten Druckverlust des Kühlers oder nach einer bestimmten Zeit wird dieser dann aus dem Produktgasstrom abgeschaltet. Das kalte Wärmeübertragungsmedium wird durch heißes verdrängt, und damit das rohe Anhydrid auf der Kühlfläche abgeschmolzen und gesammelt. Das rohe Phthalsäureanhydrid kann anschließend einer Destillation unterworfen werden.

Die Anwendung des erfindungsgemäßen Verfahrens ist besonders vorteilhaft bei Einsatz von technischem o-Xylol, das durch Extraktion oder extraktive Destillation eines aromatischen Verbindungen enthaltenden Gemisches mit Hilfe von N-Acylverbindungen erhalten worden ist. Zur Isolierung von Aromaten, wie o-Xylol, aus rohen BTX-Strömen werden gewöhnlich Extraktions- und Extraktionsdestillations-Verfahren eingesetzt. Die Extraktionsdestillation unterscheidet sich von einer normalen Destillation dadurch, dass sie in Gegenwart eines hochsiedenden Lösungsmittels durchgeführt wird, das ein hohes Lösungsvermögen für aromatische Verbindungen und ein geringes Lösungsvermögen für Paraffine und Naphthaline aufweist. Es handelt sich dabei üblicherweise um N-Acyl-Verbindungen. Das Lösungsmittel wird zweckmäßigerweise in der Nähe des Kopfs der Destillationskolonne und oberhalb des Punkts eingeleitet, an dem der BTX-Strom eingeführt wird. Die nicht-aromatischen Verbindungen werden im Allgemeinen am Kopf der Kolonne abdestilliert, und das aromatenreiche Lösungsmittel wird als Sumpfprodukt abgezogen. Die aromatischen Verbindungen können dann in einer zweiten Kolonne vom Lösungsmittel destilliert oder abgestrippt werden und das Lösungsmittel kann zurückgeführt werden. Die Auftrennung der verschiedenen aromatischen Verbindungen erfolgt zweckmäßigerweise in anschließenden Destillationsschritten. Die Verhältnisse von Lösungsmittel zu BTX-Strom liegen im Allgemeinen in der Größenordnung von 2:1 bis 6:1.

Bei der Flüssig-flüssig-Extraktion werden aromatische Verbindungen von nicht-aromatischem Material unter Verwendung eines polaren Solvens, wie N-Acyl-Verbindungen, mit hoher Selektivität für erstere extrahiert. Das Lösungsmittel wird in der Regel am Kopf des Extraktionsgefäßes aufgegeben. Es sind verschiedene Ausführungsformen des Extraktors möglich, einschließlich gepackter Säulen, Siebbodenanordnungen und ähnliche. Die rohe BTX-Fraktion wird mit Vorteil etwa in der Mitte des Extraktors eingespeist. Das nicht-aromatische Raffinat kann dann am Kopf des Reaktors abgenommen werden, während das aromatenreiche Lösungsmittel am Boden abgezogen und einer Destillationskolonne zugeführt werden kann, in der die Aromaten über Kopf abgenommen werden. Das vom Aromaten befreite Lösungsmittel kann in den Extraktor zurückgeführt werden. Der über Kopf abgenommene Aromatenstrom wird zweckmäßigerweise zur Auftrennung der einzelnen aromatischen Komponenten destilliert.

Das erfindungsgemäße Verfahren wird durch die folgenden Beispiele näher veranschaulicht:

### Beispiel:

Über mehrere Monate wurden bei 210°C und einem Druck von 1,4 bar 8400 kg o-Xylol/h in einem Luftstrom von 93.000 m³/h vermischt mit 25 kg/h gasförmigem SO₂ verdampft. Das o-Xylol hatte einen Stickstoffgehalt von ca. 1 ppm. Die Verdampferstrecke war nach diesem Zeitraum ohne Beläge.

### Vergleichsbeispiel 1:

Die Durchführung erfolgte wie Beispiel 1, jedoch hatte das o-Xylol einen Gehalt an N-Formylmorpholin von 200 ppm. Nach einer Verdampferzeit von 2 Wochen mußten aus der Verdampferstrecke ca 20 kg Rückstand entfernt werden. Dieser Rückstand hatte typischerweise folgende elementare Zusammensetzung: 54% C, 22% O, 10% N, 10% S, 4% H.

### Vergleichsbeispiel 2:

Die Durchführung erfolgte wie Vergleichsbeispiel 1, jedoch war der Luftstrom SO₂-frei. Es wurden keine Ablagerungen gefunden.

## Patentansprüche

1. Verfahren zur Herstellung von Phthalsäureanhydrid durch Oxidation von technischem o-Xylol in der Gasphase, wobei man einen o-xylol, Sauerstoff und Schwefeldioxid enthaltenden Gasstrom bei erhöhter Temperatur über einen schwermetallhaltigen Katalysator leitet, **dadurch gekennzeichnet, dass** der Gehalt des Gasstroms an N-Acyl-Verbindungen weniger als 50 ppm, bezogen auf das Gewicht an o-Xylol, beträgt.

2. Verfahren nach Anspruch 1, **dadurch gekennzeichnet, dass** man vor der Umsetzung in dem technischen o-Xylol den Gehalt an N-Acyl-Verbindungen bestimmt und,
a) wenn der Gehalt kleiner als 50 ppm ist, bezogen auf o-Xylol, das technische o-Xylol zur Umsetzung verwendet, oder
b) wenn der Gehalt größer als oder gleich 50 ppm ist, bezogen auf o-Xylol,
i) das technische o-Xylol aus dem Verfahren ausschleust, oder
ii) den Gehalt des technischen o-Xylols an N-Acyl-Verbindungen auf weniger als 50 ppm, bezogen auf o-Xylol, einstellt und das so erhaltene technische o-Xylol zur Umsetzung verwendet.

3. Verfahren nach Anspruch 2, **dadurch gekennzeichnet, dass** die Einstellung des Gehaltes an N-Acyl-Verbindungen durch Destillation erfolgt.

4. Verfähren nach Anspruch 1, 2 oder 3, **dadurch gekennzeichnet, dass** der Gehalt an N-Acyl-Verbindungen weniger als 10 ppm beträgt.

5. Verfahren nach einem der vorhergehenden Ansprüche, **dadurch gekennzeichnet, dass** man ein technisches o-Xylol verwendet, das durch Extraktion oder extraktive Destillation eines aromatische Verbindungen enthaltenden Gemisches mit Hilfe von N-Acyl-Verbindungen erhalten worden ist.

6. Verfahren nach Anspruch 5, **dadurch gekennzeichnet, dass** man als N-Acyl-Verbindung N-Formylmorpholin, N-Methylpyrrolidon und/oder N,N-Dimethylformamid, verwendet.

7. Verfahren zur Herstellung von Phthalsäureanhydrid durch Oxidation von technischem o-Xylol in der Gasphase, wobei man einen o-Xylol, Sauerstoff und Schwefeldioxid enthaltenden Gasstrom bei erhöhter Temperatur über einen schwermetallhaltigen Katalysator leitet, **dadurch gekennzeichnet, dass** der Gehalt des Gasstroms an N-Formylmorpholin weniger als 50 ppm, bezogen auf das Gewicht an o-Xylol, beträgt.

8. Verwendung von technischem o-Xylol mit einem Gehalt an N-Acyl-Verbindungen von weniger als 50 ppm zur Herstellung von Phthalsäureanhydrid durch Oxidation von o-Xylol in der Gasphase in Gegenwart von Schwefeldioxid.

## Claims

1. A process for preparing phthalic anhydride by oxidation of technical-grade o-xylene in the gas phase, in which a gas stream comprising o-xylene, oxygen and sulfur dioxide is passed at elevated temperature over a catalyst comprising heavy metals, wherein the content of N-acyl compounds in the gas stream is less than 50 ppm, based on the weight of o-xylene.

2. A process as claimed in claim 1, wherein the content of N-acyl compounds in the technical-grade o-xylene is determined prior to the reaction and
a) if the content is less than 50 ppm based on o-xylene, the technical-grade o-xylene is used for the reaction or
b) if the content is greater than or equal to 50 ppm based on o-xylene, either
i) the technical-grade o-xylene is rejected from the process or
ii) the content of n-acyl compounds in the technical-grade o-xylene is adjusted to less than 50 ppm, based on o-xylene, and the technical-grade o-xylene obtained in this way is used for the reaction.

3. A process as claimed in claim 2, wherein the content of N-acyl compounds is adjusted by distillation.

4. A process as claimed in claim 1, 2 or 3, wherein the content of N-acyl compounds is less than 10 ppm.

5. A process as claimed in any of the preceding claims, wherein the technical-grade o-xylene used is one which has been obtained by extraction or extractive distillation of a mixture comprising aromatic compounds with the aid of N-acyl compounds.

6. A process as claimed in claim 5, wherein the N-acyl compound used is N-formylmorpholine, N-methylpyrrolidone and/or N,N-dimethylformamide.

7. A process for preparing a phthalic anhydride by oxidation of technical-grade o-xylene in the gas phase, in which a gas stream comprising o-xylene, oxygen and sulfur dioxide is passed at elevated temperature over a catalyst comprising heavy metals, wherein the N-formylmorpholine content of the gas stream is less than 50 ppm, based on the weight of o-xylene.

8. The use of technical-grade o-xylene containing less than 50 ppm of N-acyl compounds for preparing phthalic anhydride by oxidation of o-xylene in the gas phase in the presence of sulfur dioxide.

## Revendications

1. Procédé de préparation d'anhydride phthalique au moyen de l'oxydation d'o-xylène technique en phase gazeuse, dans lequel on fait passer un courant gazeux contenant de l'o-xylène, de l'oxygène et du dioxyde de soufre à une température élevée sur un catalyseur contenant des métaux lourds, **caractérisé en ce que** la teneur du courant gazeux en composés N-acyle est inférieure à 50 ppm par rapport au poids de o-xylène.

2. Procédé selon la revendication 1, **caractérisé en ce que** l'on détermine la teneur en composés N-acyle dans l'o-xylène technique avant la réaction et,
a) si la teneur est inférieure à 50 ppm par rapport à l'o-xylène **en ce que** l'on utilise l'o-xylène technique pour la réaction, ou
b) si la teneur est supérieure ou égale à 50 ppm par rapport à l'o-xylène,
i) **en ce que** l'on retire le o-xylène du procédé ou,
ii) **en ce que** l'on ajuste la teneur de l'o-xylène en composés N-acyle à moins de 50 ppm par rapport à l'o-xylène et que l'on utilise l'o-xylène technique ainsi obtenu pour la réaction.

3. Procédé selon la revendication 2, **caractérisé en ce que** l'on procède à l'ajustement de la teneur en composés N-acyle au moyen d'une distillation.

4. Procédé selon l'une quelconque des revendications 1, 2 ou 3, **caractérisé en ce que** la teneur en composés N-acyle est inférieure à 10 ppm.

5. Procédé selon l'une quelconque des revendications précédentes, **caractérisé en ce que** l'on utilise un o-xylène technique que l'on a obtenu au moyen d'une extraction ou d'une distillation extractive d'un mélange contenant des composés aromatiques à l'aide de composés N-acyle.

6. Procédé selon la revendication 5, **caractérisé en ce que** l'on utilise, en tant que composé N-acyle, la N-formylmorpholine, la N-méthylpyrrolidone et/ou le N,N-diméthylformamide.

7. Procédé de préparation d'anhydride phthalique au moyen de l'oxydation d'o-xylène technique en phase gazeuse, dans lequel on fait passer un courant gazeux contenant de l'o-xylène, de l'oxygène et du dioxyde de soufre à une température élevée sur un catalyseur contenant des métaux lourds, **caractérisé en ce que** la teneur du courant gazeux en N-formylmorpholine est inférieure à 50 ppm par rapport au poids de o-xylène.

8. Utilisation d'o-xylène technique présentant une teneur en composés N-acyle inférieure à 50 ppm pour la préparation d'anhydride phthalique au moyen d'une oxydation de o-xylène en phase gazeuse en présence de dioxyde de soufre.
